# EUROPEAN PATENT APPLICATION

(11) **EP 2 201 924 A1**
(43) Date of publication of application: **30.06.2010**
(21) Application number: 08842906.3
(22) Date of filing: 15.10.2008
(51) Int. Cl.: A61J 1/00, C08L 45/00, C08L 23/08

(54) **METHOD FOR STORING THE INHALATION ANESTHETIC AND CONTAINER THEREOF**

(30) Priority: 15.10.2007 WO PCT/CN2007/070900
(71) Applicant: Jiangsu Hengrui Medicine Co., Ltd., Lianyungang, Jiangsu 222002 (CN)
(72) Inventor: JIANG, Sumei, Lianyungang Jiangsu 222002 (CN)
(74) Representative: Swinkels, Bart Willem
(86) International application number: PCT/CN2008/072687
(87) International publication number: WO 2009/052739

(57) **Abstract**

A use of a material made of cyclic olefin copolymers and graft copolymers of ethylene and octene for producing a container for storing inhalation anesthetic agents, and said container comprising a container body and a container cap, wherein the internal surface material of said container body is made of cyclic olefin copolymers and graft copolymers of ethylene and octene.

## Description

### FIELD OF THE INVENTION

The present invention relates to a use of a material made of cyclic olefin copolymers and graft copolymers of ethylene and octene for producing a container for storing anesthetic agents, and a container comprising container body and container cap, wherein the internal surface material of the container body is made of cyclic olefin copolymers and graft copolymers of ethylene and octene.

### BACKGROUND OF THE INVENTION

Sevoflurane [fluoromethyl-1,1,1,3,3,3-hexafluoroisopropyl ether or (CF₃)₂CHOCH₂F] is an inhalation anesthetic widely used, typically packaged in containers made of glass or stainless steel. However, it has been found that under certain conditions sevoflurane and the glass container may interact, thereby facilitating degradation of sevoflurane. This interaction is believed to result from the presence of Lewis acids in glass container material. Degradation of sevoflurane in the presence of Lewis acid may result in degradation products such as hydrofluoric acid.

The glass material currently used to contain sevoflurane is referred as Type III glass. This material contains silicon dioxide, calcium hydroxide, sodium hydroxide and aluminum oxide. However, the aluminum oxide contained in Type III glass tends to act as Lewis acids when exposed directly to sevoflurane, thereby facilitating degradation of sevoflurane. The degradation products, e.g., hydrofluoric acid, may etch the interior surface of the glass container, thereby exposing additional quantities of aluminum oxide to sevoflurane and thereby facilitating further degradation of sevoflurane, which will give negative impact on the product.

Efforts have been made to inhibit the degradation of sevoflurane by using containers constructed from materials that are stable and unlikely to create Lewis Acids when exposed to sevoflurane, or by using sufficient Lewis acid inhibitor. These inventions have been patented, such as Patent CN1152674C and CN1170516C, wherein Patent CN1170516C defined a container constructed from a material selected from the group consisting of polyethylene naphthalate, polymethylpentene, polypropylene, polyethylene, inomeric resins and combination thereof. These inventions can stabilize sevoflurane and inhibit the degradation of sevoflurane.

However, the packaging materials comprise but not limited to the materials described in the Patents mentioned above. With the development of technology of polymer materials, more types of polymer materials are used in packaging pharmaceutical products. Besides the materials mentioned in the above patents, some other materials were developed for storing sevoflurane. A good material named Cyclic Olefin Copolymer (COC) doesn't contain Lewis Acids which will facilitate the degradation of sevoflurane. It is not necessary to inhibit the degradation of sevoflurane with Lewis Acid inhibitor if the container is made of this material.

Cyclic Olefin Copolymer (COC) was developed by Ticona with a trade name of TOPAS. It's amorphous transparent copolymer with cyclic olefin structure. Cyclic Olefin Copolymer belongs to amorphous engineering thermoplastic, characterized by high transparency, humidity resistance, heat deflection temperature (HDT) and chemical resistance. COC possesses optical property comparable to PMMA and thermal resistance better than polycarbonate (PC). Additionally, it has dimension stability better than PMMA and PC, and other advantages such as improved steam tightness, rigidity, thermal resistance, and it is easy to be cut. TOPAS (COC) 8007 is mainly used to produce bottles for storing anesthetics and drugs. Bottles made of this material can resist all common disinfection processes, such as steam disinfection, ethylene oxide disinfection, electronic and γ-radiation sterilization, and the like. Therefore, cyclic olefin copolymer (COC) has a wide prospect in medicinal packaging. Comparing with glass or stainless steel, COC does not contain Lewis Acids such as alumina and ferric oxide, thus guarantee the stability of sevoflurane during storage.

TOPAS is highly safe and proved by US FDA. NamSA tested the samples of TOPAS (COC) 8007 following USP. Under the conditions specified in category C to H by FDA, TOPAS (COC) 8007 totally meets the requirements of FDA.

Exact^{™}Plastomers is a copolymer of ethylene and α-olefin manufactured by ExxonMobile, comprising comonomers of butylene, hexylene and octylene. It has such advantages: excellent low temperature impact resistance; low specific gravity; clean; outstanding hot sealing characteristics; good compatibility with main polymers; good flexibility and penetration resistance; excellent filling property of inorganics; good elongation and high elasticity; resistant to embrittlement and decoloration caused by radiation. Exact^{™}Plastomers (EXACT 5061) are graft copolymers of ethylene and octene (ethylene alpha octene copolymers), which meets the regulation of FDA: 21 CFR 177.1520 "olefin copolymer" (c) 3.2c and can directly contact with food materials and products. When temperature reaches thermal fill or higher than 150°F (lower than 212°F) and under the C conditions specified by 21 CFR 176.170(c) Table 2 Usage H, olefin polymers can contact with all types of food listed in Table 1 in CFR CFR 176.170(c).

In the examples, a container made of TOPAS (COC) 8007 and Exact™Plastomers (EXACT 5061) is used to store sevoflurane, in which TOPAS (COC) 8007 is the major component, whereas EXACT 5061 is used as supplementary component to increase the resistance to shatter.

### SUMMARY OF THE INVENTION

The present invention is directed to a pharmaceutical dispenser comprising: a container made of cyclic olefin copolymers and graft copolymers of ethylene and octene, wherein the container defines an interior space; and a volume of a fluoroether-containing inhalation anesthetic contained in the interior space defined by the container.

The container defining an interior space has an interior wall adjacent to the interior space. The interior wall of the container is made of cyclic olefin copolymers and graft copolymers of ethylene and octene. A predetermined volume of sevoflurane is placed in the interior space defined by the container.

The container defines an opening, and the opening provides fluid communication between the interior space and the external environment. The container has a cap to seal the opening, which is made of a material of high density polyethylene (HDPE). The cap has an interior seal gasket contacting with the fluid in said container at the top of the cap. The seal gasket is made of a material of polyethylene terephthalate (PET) membrane or polyethylene (PE) foam material. The opening defined by the container is sealed with the cap having the seal gasket. And a predetermined volume of sevoflurane is provided in the interior space defined by the container.

In the present invention, the container is used to store sevoflurane, and it can be a bottle, or in other shapes and volumes.

It has been found that containers for storing sevoflurane made of Cyclic Olefin Copolymer and graft copolymers of ethylene and octene have expected steam obstruction, chemical compatibility and strength property. Packaging materials like membrane and bottles can be obtained by common molding methods.

Containers for storing sevoflurane made of Cyclic Olefin Copolymer and graft copolymers of ethylene and octene have expected steam obstruction. And this material does not contain any Lewis Acid, thus prevent sevoflurane in the container from degrading.

An example of the Cyclic Olefin Copolymer of the present invention is Topas 8007 (COC) provided by Ticona (USA), wherein 8007 represents a class level, which is acknowledged by technicians in this filed. Other class level of Cyclic Olefin Copolymer can be used within the range specified by the present patent application.

Graft copolymers of ethylene and octene (ethylene alpha octene copolymers) used in the present invention are Exact™Plastomers (EXACT 5061) manufactured by Exxon Mobil.

Sevoflurane is an inhalant, comparatively volatile solution. The degradation products of sevoflurane, e.g., hydrofluoric acid etc., are hazard to human body. Therefore, besides the high impurity of sevoflurane, the container should guarantee the stability of solution of sevoflurane. In the present invention, purity of sevoflurane is higher than 99.95wt%, preferably higher than 99.98wt%, calculated on anhydrous basis.

The advantage of the present invention is that sevoflurane will be stable when stored in the container made of TOPAS (COC) and Exact™Plastomers (EXACT 5061), even if under a temperature of 40°C.

The water content of the sevoflurane of the present invention is kept at 150 ppm to 1400 ppm by packing or storing the sevoflurane in a container made of TOPAS (COC) 8007 and Exact™Plastomers (EXACT 5061). In another example, the water content is below 140 ppm. The water content is determined by Karl-Ficher method. The purity of sevoflurane remains above 99.95wt% or even up to 99.98wt% after storing the sevoflurane in the container under atmospheric temperature for 6 months, or under 40°C, relative humidity of 20% for 6 months. Sevoflurane will not degrade if packaged or stored in the container made of TOPAS (COC) 8007 and Exact™Plastomers (EXACT 5061) of the present invention.

The present invention is further illustrated by the following examples which are presented for the purpose of demonstrating, but not limiting, the method of this invention.

### PREFERRED EMBODIMENTS

### EXAMPLES

### Example 1

This example describes the stability of sevoflurane (water content: 150 ppm-1400 ppm) stored in a container made of TOPAS (COC) 8007 and Exact™Plastomers (EXACT 5061). In an experiment performed in January, 2008, the water content of sevoflurane was determined to be 165 ppm by Karl-Ficher method. Place a sample of sevoflurane in a container made of TOPAS (COC) 8007 and Exact^{™}Plastomers (EXACT 5061) previously dried for 2 hours under 105°C, seal and store under a temperature of 40°C, a relative humidity of 75% for 6 months. Then the purity of the sevoflurane is determined by gas chromatography, and a result of 99.959 wt% is obtained, which indicates that there is no degradation.

### Example 2

This example describes the stability of sevoflurane (water content: 150 ppm-1400 ppm) stored in a container made of TOPAS (COC) 6013 and Exact™Plastomers (EXACT 5061). In an experiment performed in January, 2008, the water content of sevoflurane was determined to be 1300 ppm by Karl-Ficher method. Place a sample of sevoflurane in a container made of TOPAS (COC) 8007 and Exact™Plastomers (EXACT 5061) previously dried for 2 hours under 105°C, seal and store under a temperature of 40°C, a relative humidity of 75% for 6 months. Then the purity of the sevoflurane is determined by gas chromatography, and a result of 99.964 wt% is obtained, which indicates that there is no degradation.

### Example 3

This example describes the stability of sevoflurane (water content: less than 140 ppm) stored in a container made of TOPAS (COC) 8007 and Exact™Plastomers (EXACT 5061). In an experiment performed in January, 2008, the water content of sevoflurane was determined to be 80 ppm by Karl-Ficher method. Place a sample of sevoflurane in a container made of TOPAS (COC) 8007 and Exact™Plastomers (EXACT 5061) previously dried for 2 hours under 105°C, seal and store under a temperature of 40°C, a relative humidity of 75% for 6 months. Then the purity of the sevoflurane is determined by gas chromatography, and a result of 99.959 wt% is obtained, which indicates that there is no degradation.

### Example 4

Data in the following table justify the stability of sevoflurane stored long-term in container made of TOPAS (COC) 8007 and Exact^{™}Plastomers (EXACT 5061). Water content of sevoflurane was calculated by Karl Fischer method. Place the sevoflurane in a 250 mL container made of TOPAS (COC) 8007 and Exact^{™}lastomers (EXACT 5061), and perform stability test under certain conditions or ambient temperature. In all the tests, the purity of the sevoflurane (determined by gas chromatography) indicates that there is no degradation of sevoflurane.

| Container type | Manufacturing date | Initial moisture | Duration | Conditions | Final purity | Result |
|---|---|---|---|---|---|---|
| 250ml | 2008.01 | 90 ppm | 180 days | 25°C/ 60% (RH) | 99.971% | No degradation |
| | 2008.01 | 170 ppm | 180 days | 25°C/ 60% (RH) | 99.964% | No degradation |
| | 2008.01 | 1100 ppm | 180 days | 25°C/ 60% (RH) | 99.977% | No degradation |

Examples are described in detail for the purpose of demonstrating, but not limiting, the method of this invention, which should be understood by technicians in this field. Any changes based on the present invention are covered by this patent.

## Claims

1. A use of a material made of cyclic olefin copolymers and graft copolymers of ethylene and octene in manufacturing a container for storing anesthetic agents.

2. The use of the material according to Claim 1, wherein the anesthetic agent is fluoroether inhalation anesthetics.

3. The use of the material according to Claim 2, wherein the fluoroether is sevoflurane.

4. A container comprising a container body and a container cap, **characterized in that** the internal surface material of the container body is made of cyclic olefin copolymers and graft copolymers of ethylene and octene.

5. The container of Claim 4, wherein the container cap is equipped with a seal gasket.

6. The container of Claim 4 or 5, wherein the material of the cap is high density polyethylene.

7. The container of Claim 5, wherein the material of said seal gasket is polyethylene terephthalate membrane or polyethylene foam material.

8. A pharmaceutical dispenser comprising:
a container made of cyclic olefin copolymers and graft copolymers of ethylene and octene, wherein the container defines an interior space; and a volume of a fluoroether-containing inhalation anesthetic contained in the interior space defined by the container.

9. The pharmaceutical dispenser of Claim 8, wherein the fluoroether inhalation anesthetic is sevoflurane.

10. The pharmaceutical dispenser of Claim 8, wherein said container defines an opening therein, said opening provides fluid communication between said interior space defined by said container and an external environment of said container; said pharmaceutical dispenser further comprises a cap equipped with a seal gasket to seal said opening of said container, said cap is made of a material of high density polyethylene.

11. The pharmaceutical dispenser of Claim 8, wherein said container defines an opening therein, said opening provides fluid communication between said interior space defined by said container and an external environment of said container, said pharmaceutical dispenser further comprises a cap having an interior seal gasket that contacts with the fluid in said container at the top of the cap, said seal gasket of said cap is made of a material of polyethylene terephthalate membrane or polyethylene foam material.

12. A pharmaceutical dispenser comprising:
a container defining an interior space, wherein said container has an interior surface adjacent to said interior space, and said interior surface is made of cyclic olefin copolymers and graft copolymers of ethylene and octene; and a predetermined volume of sevoflurane in said interior space of said container.

13. A method for storing sevoflurane as inhalation anesthetic agent comprising the steps of:
providing a predetermined volume of sevoflurane as inhalation anesthetic agent;
providing a container defining an interior space, wherein said container is made of cyclic olefin copolymers and graft copolymers of ethylene and octene; and
providing said predetermined volume of sevoflurane in said interior space defined by said container.

14. The method for storing sevoflurane as inhalation anesthetic agent of Claim 13, wherein said container defines an opening therein to provide fluid communication between said interior space defined by said container and external environment of said container, said method further comprises the steps of:
providing a cap made of high density polyethylene to seal said opening defined by said container.

15. The method for storing sevoflurane as inhalation anesthetic agent of Claim 13, wherein said container defines an opening therein to provide fluid communication between said interior space defined by said container and an external environment of said container; said method further comprises the steps of
providing a cap constructed to seal said opening defined by said container, said cap has an interior seal gasket that contacts with the fluid in said container at the top of the cap, said seal gasket is made of cyclic olefin copolymers and graft copolymers of ethylene and octene; and
providing said cap having said interior seal gasket to seal said opening defined by said container.

16. A method for storing sevoflurane as inhalation anesthetic agent comprising the steps of
providing a predetermined volume of sevoflurane as inhalation anesthetic agent; providing a container defining an interior space, wherein said container has an interior wall adjacent to said interior space defined by said container, said interior wall of said container is made of cyclic olefin copolymers and graft copolymers of ethylene and octene; and
providing said predetermined volume of sevoflurane in said interior space defined by said container.

17. The method for storing sevoflurane as inhalation anesthetic agent of Claim 16, wherein said container defines an opening to provide fluid communication between said interior space defined by said container and an external environment of said container, said method further comprises the steps of:
providing a cap made of high density polyethylene to seal said opening defined by said container.

18. The method for storing sevoflurane as inhalation anesthetic agent of Claim 16, wherein a cap having a seal gasket is provided to seal said opening defined by said container, said seal gasket contacts with the fluid in said container at the top of the cap, said seal gasket is made of cyclic olefin copolymers and graft copolymers of ethylene and octene, and said cap having said seal gasket is provided to seal said opening defined by said container.
